# EUROPEAN PATENT SPECIFICATION

(11) **EP 1 757 186 B1**
(45) Date of publication and mention of the grant of the patent: **05.10.2011**
(21) Application number: 05743686.7
(22) Date of filing: 25.05.2005
(51) Int. Cl.: A01K 67/027

(54) **RESTLESS LEGS SYNDROME/PERIODIC APPENDICULAR DYSKINESIA MODEL ANIMAL**
MODELLTIER FÜR EKBOM-SYNDROM/PERIODISCH AUFTRETENDE BLINDDARMDYSKINESE
ANIMAL MODELE DE DYSKINESIE APPENDICULAIRE PERIODIQUE/SYNDROME DES JAMBES SANS REPOS

(30) Priority: 31.05.2004 JP 2004162468
(43) Date of publication of application: 28.02.2007
(73) Proprietor: Japan Science and Technology Agency, Kawaguchi-shi, Saitama 332-0012 (JP)
(72) Inventor: KADOTANI, Hiroshi, Kamigyo-ku, Kyoto 602-0915 (JP)
(74) Representative: Behnisch, Werner
(86) International application number: PCT/JP2005/009517
(87) International publication number: WO 2005/115134

(56) References cited:
- JP-A- 2002 544 474
- BAIER PC, WINKELMANN J, HÖHNE A, LANCEL M, TRENKWALDER C.: "Assessment of spontaneously occurring periodic limb movements in sleep in the rat." NEUROL SCI., vol. 198, no. 1, 15 June 2002 (2002-06-15) , pages 71-77, XP002575497
- ESTEVES AM, DE MELLO MT, LANCELLOTTI CL, NATAL CL, TUFIK S.: "Occurrence of limb movement during sleep in rats with spinal cord injury." BRAIN RES., vol. 1017, no. 1-2, 20 June 2004 (2004-06-20), pages 32-34, XP002575498
- CLEMENS S, HOCHMAN S.: "Conversion of the modulatory actions of dopamine on spinal reflexes from depression to facilitation in D3 receptor knock-out mice." J NEUROSCI., vol. 24, no. 50, 15 December 2004 (2004-12-15), pages 11337-11345, XP002575499
- BAIER PC, ONDO WG, WINKELMANN J.: "Animal studies in restless legs syndrome." MOV DISORD., vol. 22, no. suppl 18, 26 June 2007 (2007-06-26), pages S459-S465, XP002575500
- ONDO WG, ZHAO HR, LE WD.: "Animal models of restless legs syndrome." SLEEP MED., vol. 8, no. 4, 30 April 2007 (2007-04-30), pages 344-348, XP002575501
- MANCONI M, HUTCHINS W, FEROAH TR, ZUCCONI M, FERINI-STRAMBI L.: "On the pathway of an animal model for restless legs syndrome..." NEUROL SCI, vol. 28, 1 January 2007 (2007-01-01), XP002575502
- TAKAKI J. ET AL.: 'Clinical and psychological aspects of restless legs syndrome inuremic patients on hemodialysis.' AM.J. KIDNEY DIS. vol. 41, no. 4, 2003, pages 833 - 839, XP002990346
- COLLADO-SEIDEL V. ET AL.: 'Clinical and biochemical findings in uremic patients with and without restless legs syndrome.' AM.J.KIDNEY DIS. vol. 31, no. 2, 1998, pages 324 - 328, XP002990347

## Description

### Technical Field

The present invention relates to i) a model rodent of Restless Legs Syndrome (RLS) and Periodic Limb Movement Disorder (PLMD), and ii) a method for production of the model rodent that may be used in a method for identification of a causative substance of RLS and PLMD by use of the model animal, etc.

### Background Art

Restless Legs Syndrome (RLS) is such a frequent disease or disorder that several percent of ordinary people are suffering from it. Its symptom includes the following various symptoms; i) RLS patient is likely to be driven by strong impulse that he/she wants to move his/her leg, ii) he/she often feels his/her leg tickled, iii) he/she feels as if some insect crept on his/her leg, iv) such symptoms especially happen when he/she is motionless and the symptoms can be reduced by moving his/her leg, v) the symptoms deteriorate especially when he/she is in bed from evening to night, resulting in sleep problem. The cause of RLS is unclear, but it is reported that 63-92% of RLS patients have another RLS patient in their family. It is also said that RLS will often deteriorate by caffeine or antidepressant drug.

Moreover, 80-90% of RLS patients are suffering from Periodic Limb Movement Disorder (PLMD). As mentioned above, RLS patient is likely to feel his/her leg tickled especially when he/she is in bed at night, resulting in a symptom of sleep trouble having difficulty in sleeping. On the other hand, PLMD patient shows a symptom that he/she repeats the movement of his/her limbs while sleeping at a cycle of 5-90 seconds and such movement continues for a while, and stops for a while. Both symptoms of RLS and PLMD very often appear together in the same patient and thus, there is a high possibility that these two diseases develop by the same reason.

RLS and PLMD develop especially in the renal failure patients and the pregnant women. The symptoms of RLS and PLMD are recognized in 1/3 or more of the dialysis patients with renal failure (see References (1)-(3) below, concerning research results of RLS in the renal failure patients.)

References
(1) Y. Oka, S. Koike, K. Yamamoto, H. Kadotani and Y. Inoue, "Investigation of the Restless Legs Syndrome in the dialysis patients with renal failure." program and abstracts of the 19th meeting of insomnia research society, page 32;
(2) TAKAKI J. ET AL.: "Clinical and psychological aspects of restless legs syndrome inuremic patients on hemodialysis." AM.J. KIDNEY DIS., vol. 41, no. 4, 2003 , pages 833-839,
(3) COLLADO-SEIDEL V. ET AL.: "Clinical and biochemical findings in uremic patients with and without restless legs syndrome." AM.J.KIDNEY DIS., vol. 31, no. 2, 1998 , pages 324-328. References (2) and (3) respectively describe analyzing components of a patient group and non patient group of the RLS and comparing both groups to further study components found to be significantly different. Though it is understood that certain blood components may have something to do with the RLS, no particular causative agent has been specified and no animal model was established or suggested. Periodic hindlimb movements (PHLM) in sleep can occur spontaneously in rats and were observed to validate whether the observed PHLM constitute a good model for human PLMS or even RLS ((4) Baier PC, et al. J Neurol Sci. 2002 Jun 15;198(1-2):71-77. A clear effect of age on this phenomenon was seen, with only old animals displaying PHLM.

### Disclosure of the Invention

### [Problem to be solved by the Invention]

Many dialysis patients with renal failure are suffering from sleep trouble, and their QOL (Quality of Life) is remarkably damaged for that. The main reason is Restless Legs Syndrome (RLS) and Periodic Limb Movement Disorder (PLMD). Therefore, it is desirable for such renal failure patients to promptly identify a causative substance of RLS and PLMD, and further to develop and establish a more objective diagnostic method and therapy for RLS and PLMD. For this purpose, it is very effective to firstly produce a model animal of RLS and PLMD, to identify a causative substance of these diseases by use of the model animal, and also to develop and evaluate a diagnostic method and therapy by use of it.

As mentioned above, RLS and PLMD are diseases which very often cause the sleep problem. Additionally, RLS and PLMD are diseases which may be related to ADHD (Attention Deficit Hyperactivity Disorder) etc that is now a matter of social concern. By producing a model animal of RLS and PLMD, the model animal could be also used to develop and evaluate a diagnostic method and therapy for such other related diseases.

The object of the present invention is to solve the above-mentioned problems and provide a model rodent of Restless Legs Syndrome (RLS) and Periodic Limb Movement Disorder (PLMD), as well as a method for producing the model animal useful in a method for identifying a causative substance of these diseases by use of the model animal, etc

### [Means for solving the problem]

The inventor paid attention to the fact that RLS and PLMD develop especially in the renal failure patients and the pregnant women, as mentioned above, and set up a hypothesis that the reason may be decrease in excretion of causative substances from the kidney in the renal failure patients, and in the pregnant women, the reason may be overproduction of metabolites while pregnancy. Based on this hypothesis, the inventor parenterrally administered a part of peripheral blood prepared from the patient to a laboratory animal and found the rodent showing the symptom of RLS and PLMD. Thus, the inventor succeeded in the production of a model rodent in which the symptom of RLS and PLMD was well induced or reproduced, which led to the present invention.

That is, the present invention includes the following industrially and medically useful inventions of A) to F).
A) A method for producing a model a rodent of Restless Legs Syndrome (RLS) and Periodic Limb Movement Disorder (PLMD), characterized by administering parenterally to an rodent, plasma constituents in blood prepared from a dialysis patient with renal failure who shows the symptom of Restless Legs Syndrome (RLS) and Periodic Limb Movement Disorder (PLMD). .
   There is a high possibility that the urine of RLS and PLMD patient with normal renal function includes a causative substance, which is identical to that in the plasma of the patient, or its metabolite. Therefore, even by administering the urine of the patient to an animal, the symptom of RLS and PLMD might be shown in the animal, as shown by administering the plasma of the patient.
B) A method for producing a model rodent according to the above A), characterized by administering through intraperitoneal injection to a rodent the plasma constituents in blood.
C) A model rodent of Restless Legs Syndrome (RLS) and Periodic Limb Movement Disorder (PLMD), produced by a method according to the above A) or B).
D) A model rodent according to the above C), characterized in that the rodent is a mouse
E) Further is described a method for identifying a causative substance of Restless Legs Syndrome A (RLS) and Periodic Limb Movement Disorder (PLMD), characterized by using a model rodent according to the above C) or D).
F) A method for developing a diagnostic method and therapy for Restless Legs Syndrome (RLS) and Periodic Limb Movement Disorder (PLMD), characterized by using a model rodent according to the above C) or D), or by using as a target a causative substance identified by use of the model animal.

### [Effect of the Invention]

A model rodent of the present invention is a firstly produced model rodent of Restless Legs Syndrome (RLS) and Periodic Limb Movement Disorder (PLMD). The model rodent can be easily produced, and can be used to identify a causative substance of RLS and PLMD, and also to develop and evaluate a diagnostic method and therapy for RLS and PLMD.

Although the concrete use of the present invention is described later, the present invention can be used in various aspects such as i) identification of a causative substance in blood (or urine) collected from RLS and PLMD patients, ii) examination as to whether or not a candidate is really a causative substance of RLS and PLMD, iii) development of a new therapy for the removal of causative substances, iv) development of a new dialysis machine having such removal function, v) development of a new diagnostic method or tool for the measurement or quantification of causative substances in a sample, vi) examination as to whether or not a candidate really has a therapeutic effect for RLS and PLMD, and vii) development and evaluation of a diagnostic method and therapy for other related diseases such as ADHD.

### Brief Description of the Drawings

Figure 1 is a graph showing a result of behavior observation of model mice of the present invention and other mice used in experiments.
Figure 2 shows one of images of behavior of a model mouse of the present invention, as compared with that of a control mouse.
Figure 3 is a graph showing that mice showed the symptom of RLS and PLMD even when the plasma constituents from RLS and PLMD patients with normal renal function were administered to the mice.

### Best Mode for Carrying Out the Invention

Specific embodiments of the present invention are described in detail below.

### [1] Model rodent of the present invention and method for its production

As mentioned above, a model rodent of the present invention is a model rodent of Restless Legs Syndrome (RLS) and Periodic Limb Movement Disorder (PLMD), produced by administering parenterally to a rodent, plasma constituents in blood prepared from a dialysis patient with renal failure who shows the symptom of Restless Legs Syndrome (RLS) and Periodic Limb Movement Disorder (PLMD).

The inventor paid attention to the fact that RLS and PLMD were found especially in renal failure patients, and that the symptom was able to be ameliorated by dialysis (especially, by long time dialysis). Therefore, the inventor set up a hypothesis that the diseases might be due to accumulation of substances to be excreted from the kidney, and also thought that the symptom of RLS and PLMD might be induced or reproduced by administering to a laboratory animal the blood prepared from the patient.

In order to test this hypothesis, peripheral blood was collected from renal failure patients in whom RLS and PLMD were diagnosed, and its plasma constituents were administered to a mouse by intraperitoneal injection. The result was that the mouse started to take the action pathognomonic in these disorders. On the other hand, such action was not observed when the plasma constituents from a healthy person developing neither RLS nor PLMD were administered. This result suggested that a causative substance exists in the patient's peripheral blood, and its physiological activity can be examined by using a laboratory animal such as a mouse.

Thus, a model rodent of the present invention shows the symptom of RLS and PLMD, and it is the model rodent of RLS and PLMD produced for the first time. This model rodent can be used to identify a causative substance of RLS and PLMD and other associated diseases.

The following is a more detailed description of a method of making a model rodent of the present invention.

### (1) Preparation of plasma constituents

First, peripheral blood is collected from one or plural patients showing the symptom of RLS and PLMD. The blood-collecting method is not especially limited. For example, the method using a blood-collecting tube with anticoagulant can be adopted, as adopted in the example described later. After collection of a blood sample, its cell constituents such as red blood cells and white blood cells are removed by centrifugal separation etc. Thus produced plasma constituents are frozen for preservation in a freezer etc until its use. The level of fractionation is not necessary to be so strict, and some cell constituents such as platelets may be included in the plasma constituents.

### (2) Dosage volume and method of administration

The dosage volume of the plasma constituents administered to a target animal may be determined in consideration of various factors such as the kind of a used laboratory animal, its weight and size, method of administration, a smallest volume necessary for showing the symptom, and undesired influence due to overdosing. For example, when an adult mouse is used as the laboratory animal, the dosage volume may be set to be within about 0.1-2.0 mL.

The method of parenteral administration is, including intravenous administration, intramuscular administration and subcutaneous administration, in addition to intraperitoneal administration. When the dosage volume is large, the plasma constituents may be administered separately into twice or more times.

When the urine constituents from the patient are administered, it is preferable to concentrate it, if necessary, with fractionation. Concentration can be carried out with concentration column, centrifugal ultrafiltration, gel filtration chromatography, solvent absorption concentration, centrifugal concentration device, etc. In the case of administration of the urine constituents, the dosage volume and method of administration can be determined, as well as the case of administration of the plasma constituents.

### (3) Kind of animal

The kind of non human animal that may be used as a model animal is not especially limited, as long as it can be used as the laboratory animal. The following mammals are examples of model animal; the bos taurus, the pig, the sheep, the goat, the rabbit, the dog, the cat, the guinea pig, the hamster, the mouse and the rat. Among these, the rodent such as the mouse and the rat is more preferable, since it is easily available and more frequently used as the laboratory animal. In the following example, model mice of the present invention were produced by intraperitoneal administration of the plasma constituents in blood collected from RLS and PLMD patients.

### (4) Method for identifying a causative substance

The model rodent of the present invention can be used to identify a causative substance of RLS and PLMD.

For example, blood samples collected from RLS and PLMD patients are administered to laboratory animals after different fractionations with various methods, in order to compare appearance of the symptom and the degree of severeness, etc. By such comparative study, it is possible to identify not only a fraction including a causative substance but also a causative substance itself. The causative substance can be analyzed more in detail by administering to rodents, candidate causative substances found by such method (or, another method), followed by observation of appearance of the symptom and the degree of severeness, etc. The model rodent of the present invention can be used as a control animal in such research analysis. The model rodent of the present invention can be also used to identify a causative substance by comparing the amount of candidate causative substances in the blood etc of the model rodent, with that of a control rodent.

Here, one example will be described as a method for identifying a causative substance. First, patient's plasma constituents (or, urinary constituents) are separated by a method selected from the fractionation according to molecular weight using centrifugal ultrafiltration unit or gel filtration, the fractionation according to hydrophobicity using reverse phase column chromatography or hydrophobic chromatography, the fractionation according to electric charge using ion exchange chromatography or isoelectric chromatography, and the fractionation according to affinity using affinity chromatography, etc. Afterwards, it can be determined which fraction contains a causative substance, by using a laboratory animal such as the mouse. The one or two-dimensional electrophoresis of the determined fraction derived from the patient may be carried out together with a fraction derived from healthy person, followed by mass spectroscopy of bands or spots (in the two-dimensional electrophoresis) according to the difference of the amount (density), to identify a causative substance.

The identification of a causative substance of RLS and PLMD can contribute to the development of a diagnostic method and therapy for RLS and PLMD.

For example, since many dialysis patients with renal failure are suffering from RLS and PLMD, enormous therapeutic effect can be expected by simultaneously removing the causative substance at the time of artificial dialysis. The present invention can be used to develop new dialysis equipment with such therapeutic effect. Of course, the model rodent of the present invention can be used at the development stage of a new therapeutic drug. For example, the model rodent of the present invention can be used for evaluation of the therapeutic effect of a candidate drug by administering it to the model animal and examining as to whether or not the symptom can be ameliorated.

As to the development of a diagnostic method, it is possible to provide a more objective new diagnostic method for RLS and PLMD, for example by the development of a kit for the measurement and quantification of the amount of causative substances in blood etc of a subject. Conventionally, the diagnosis of RLS and PLMD had been based on a subjective symptom, and the development of a more objective diagnostic method had been strongly desired. The present invention can be used for such development of a more objective diagnostic method and its evaluation, as well as the development and evaluation of a new diagnostic tool.

Additionally, RLS and PLMD are diseases which may be related to ADHD (Attention Deficit Hyperactivity Disorder) etc that are becoming an issue in school education and a matter of social concern. By producing a model rodent of RLS and PLMD and identifying a causative substance, the model rodent could be also used to develop and evaluate a diagnostic method and therapy for such other related diseases.

### [2] Example of making a model rodent of the present invention (Example)

The present invention is described in detail below through its Example producing a model mouse of RLS and PLMD, but in no way is the present invention limited to this Example.

### [Concrete experimental procedure for production of a model mouse]

First of all, peripheral blood was collected from dialysis patients with renal failure, showing the symptom of RLS and PLMD, and diagnosed as 'RLS+.' More specifically, peripheral blood was collected from the patients by use of the 4.5ml blood-collecting tube with 3.8% sodium citrate buffer as an anticoagulant. Peripheral blood was also collected, as the control, from not only healthy persons but also dialysis patients with renal failure, showing the symptom of neither RLS nor PLMD, and hence diagnosed as 'RLS-.'

After collection, the blood-collecting tube was immediately placed on the ice, followed by centrifugal separation of 3,500 rpm for ten minutes. Thus obtained supernatants (plasma constituents) were separated into several containers, for frozen storage using the -80°C freezer or the dry ice.

Afterwards, the plasma constituents were defrosted from the frozen storage, and they were administered to each mouse by intraperitoneal (IP) injection. Altenatively, supernatants of them were administered to each mouse after fractionation. The method of fractionation was selected from the fractionation according to molecular weight using centrifugal ultrafiltration unit or gel filtration, the fractionation according to hydrophobicity using reverse phase column chromatography or hydrophobic chromatography, the fractionation according to electric charge using ion exchange chromatography or isoelectric chromatography, and the fractionation according to affinity using affinity chromatography. The plasma constituents administered to each mouse were one of i) the plasma constituents from the patients diagnosed as 'RLS+', ii) the plasma constituents from the dialysis patients diagnosed as 'RLS-', and iii) the plasma constituents from the healthy persons. The dosage volume of the plasma constituents was set to be within 0.1-2.0mL. Moreover, a saline, or a buffer, was administered to another mouse group, as the control, by intraperitoneal injection. The intraperitoneal injection was carried out under anesthetization by ether or barbiturate.

Before the intraperitoneal administration of the plasma constituents etc to each mouse, the behavior and action of each mouse was observed with a video, and the observation was continued for about one hour after the administration. Based on thus obtained images with the video, the frequency of overextension of lower limbs in ten minutes (which were 10-20 minutes after the intraperitoneal injection) was counted in each mouse.

### [Result of behavior observation of the model mouse]

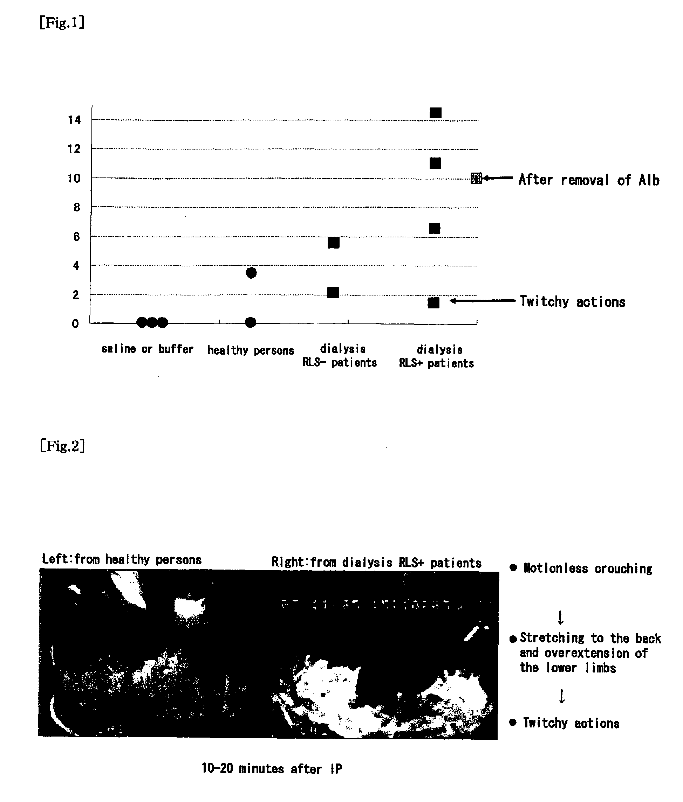

Fig. 1 is a graph showing the result of behavior observation of each mouse. The vertical axis represents the frequency of overextension of lower limbs in the above-mentioned ten minutes, and the signs of rounds and squares in the graph represent the result of each mouse. As shown in the Figure, no overextension of lower limbs was observed in the mice having the saline or buffer administered. In these mice, no change of the action was observed. Also, the change of the action was hardly observed in not only the mice having the administered plasma constituents from the healthy persons, but also the mice having the administered plasma constituents from the (RLS-) dialysis patients with neither RLS nor PLMD.

In contrast, the frequency of overextension of lower limbs remarkably increased in the mice having the administered plasma constituents from the (RLS+) dialysis patients with RLS and PLMD. The change of the action was also remarkably observed in these mice.

Moreover, another sample, in which albumin had been removed from the plasma constituents of the (RLS+) dialysis patients, was administered to a mouse (see "After removal of Alb" in the Figure). The activity eliciting the overextension of mouse's lower limbs was also observed in this sample.

Figure 2 shows, on the right side, one of images of behavior of the mouse, to which the plasma constituents of the (RLS+) dialysis patient were administered. For comparison, one of images of behavior of the control mouse (to which the plasma constituents of the healthy person were administered) is also shown on the left side of the Figure. In the mouse to which the plasma constituents of the (RLS+) dialysis patient were administered, the following behavior and action were observed such as i) motionless crouching, ii) stretching to the back and overextension of the lower limbs, and iii) twitchy actions. These behavior and action very closely resembled the symptoms of RLS and PLMD. Thus, the mouse can be used as a model mouse of these disorders.

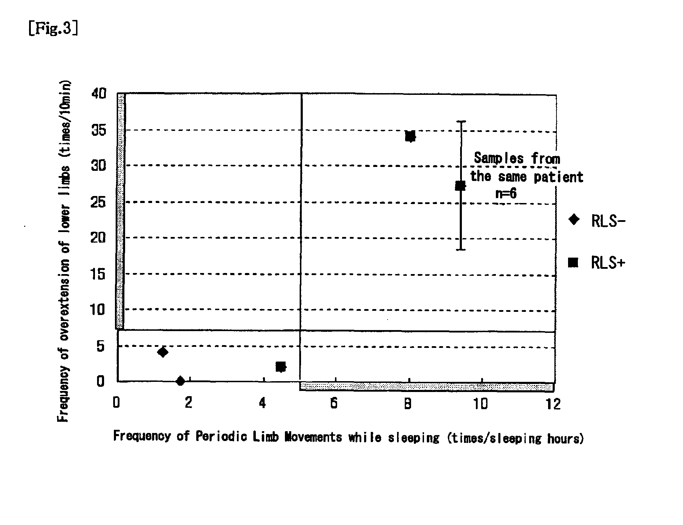

Furthermore, the plasma constituents from RLS and PLMD patients with normal renal function were administered to mice. Fig. 3 shows the result.

In the graph of Fig. 3, the signs of diamonds and squares represent the result of each mouse. The signs of diamonds represent the result of intraperitoneal administration of plasma constituents from healthy persons (RLS-) with normal renal function, whereas the signs of squares represent the result of intraperitoneal administration of plasma constituents from RLS and PLMD patients (RLS+) with normal renal function. The horizontal axis represents the frequency of Periodic Limb Movements while sleeping (times / sleeping hours), while the vertical axis represents the frequency of overextension of lower limbs in the ten minutes, as well as the vertical axis of Fig. 1.

As shown in Fig. 3, the mice showed the action or symptom pathognomonic in RLS and PLMD, even by administering the plasma constituents from the RLS and PLMD patients with normal renal function to the mice. In samples collected from the same patient, reproducibility of the symptom was high, regardless of the blood-collecting period or the experiment date (the number of experiments is n=6). These results show that a causative substance also exists in the plasma of the RLS and PLMD patients with normal renal function.

In the above-mentioned experiment results, the mice showed more pathognomonic actions by administering the plasma constituents from the RLS+ patients with normal renal function, as compared with the administration of the plasma constituents from the RLS+ patients with renal failure. Thus produced model mouse of RLS and PLMD can be useful for the development of a diagnostic method and therapy of RLS accompanied with PLMD.

### Industrial Applicability

Thus, the present invention relates to a model rodent of RLS and PLMD, and it can be used for not only the identification of a causative substance of RLS and PLMD but also the development and evaluation of a diagnostic method and therapy for these diseases. The present invention also has other various industrial utilities as mentioned above.

## Claims

1. A method for producing a model rodent of Restless Legs Syndrome (RLS) and Periodic Limb Movement Disorder (PLMD), **characterized by** administering parenterally to a rodent, plasma constituents in blood prepared from a dialysis patient with renal failure who shows the symptom of Restless Legs Syndrome (RLS) and Periodic Limb Movement Disorder (PLMD).

2. A method for producing a model rodent according to claim 1, **characterized by** administering through intraperitoneal injection to a rodent the plasma constituents in blood.

3. A model rodent of Restless Legs Syndrome (RLS) and Periodic Limb Movement Disorder (PLMD), produced by a method according to claim 1 or 2.

4. A model rodent according to claim 3, **characterized in that** the rodent is a mouse.

## Patentansprüche

1. Verfahren zur Herstellung eines Modellnagetiers für das Restless Legs Syndrome (RLS) und die periodische Gliedmaßenbewegungsstörung ("Periodic Limb Movement Disorder") (PLMD), charakterisiert durch parenterale Verabreichung von Plasmabestandteilen im Blut, das von einem Dialysepatienten mit Nierenversagen, der die Symptome von Restless Legs Syndrome (RLS) und der periodischen Gliedmaßenbewegungsstörung ("Periodic Limb Movement Disorder") (PLMD) zeigt, hergestellt wurde, an ein Nagetier.

2. Verfahren zur Herstellung eines Modellnagetiers gemäß Anspruch 1, charakterisiert durch Verabreichung der Plasmabestandteile im Blut an das Nagetier durch intraperitoneale Injektion.

3. Modellnagetier für das Restless Legs Syndrome (RLS) und die periodischen Gliedmaßenbewegungsstörung ("Periodic Limb Movement Disorder") (PLMD), hergestellt durch ein Verfahren gemäß Anspruch 1 oder 2.

4. Modellnagetier gemäß Anspruch 3, charakterisiert **dadurch**, dass das Nagetier eine Maus ist.

## Revendications

1. Procédé de production d'un modèle rongeur du syndrome des jambes sans repos (SJSR) et de la myoclonie nocturne (MIMS), **caractérisé par** l'administration parentérale à un rongeur de composants de plasma dans le sang préparés à partir d'un patient dialysé souffrant d'une insuffisance rénale qui présente le symptôme du syndrome des jambes sans repos (SJSR) et de la myoclonie nocturne (MIMS).

2. Procédé de production d'un modèle rongeur selon la revendication 1, **caractérisé par** l'administration par injection intrapéritonéale à un rongeur de composants de plasma dans le sang.

3. Modèle rongeur du syndrome des jambes sans repos (SJSR) et de la myoclonie nocturne (MIMS), produit selon le procédé selon la revendication 1 ou 2.

4. Modèle rongeur selon la revendication 3, **caractérisé par le fait que** le rongeur est une souris.
